# EUROPEAN PATENT APPLICATION

(11) **EP 1 088 489 A2**
(43) Date of publication of application: **04.04.2001**
(21) Application number: 00301319.0
(22) Date of filing: 21.02.2000
(51) Int. Cl.: A43B 17/10, A61M 35/00

(54) **A shoe equipped with medical treatment for athlete's foot and bag body containing medicine for athlete's foot**

(30) Priority: 29.09.1999 JP 27698499
(71) Applicant: Ohki, Masayuki, Kamagaya-shi, Chiba-ken (JP); Kabushiki Kaisha Yokote Japan, Yokote-shi, Akita-ken (JP)
(72) Inventor: Mori, Hirohisa, Ever Life Kukoh Higashi No.212, Kasuya-Gun, Fukuoka-Ken (JP); Fujishima, Shuji, Matsudo-shi, Chiba-Ken (JP)
(74) Representative: Mosey, Stephen George

(57) **Abstract**

A bag body (1) containing medicine (3) for athlete's foot is placed inside a shoe, so that when a person who is suffering from athlete's foot wears the shoe the medicine (3) is dispersed into the shoe and treats his athlete's foot during walking. A sterilizing agent and/or a deodorizing agent can be included with the medicine (3), in order to sterilize various bacteria and viruses and prevent offensive foot odors.

## Description

In the past, no effective or reliable treatment existed for athlete's foot. A person suffering from athlete's foot can wear well-ventilated shoes, but may find this has little effect on the disease. In this situation, a person may apply medicine as soon as he takes off his shoes, but it may take a long time to treat athlete's foot by this method. Shoes worn at the office are normally airtight, and cause the foot to perspire, and as a result the moisture and heat in the shoe may increase the number of bacteria or viruses on the wearer's feet, leading to athlete's foot and malodorous feet. Accordingly, a person with this condition may try to avoid the same by changing into an open or meshed sandal at the office. An extra, or separate, insole has been developed to improve these conditions, but this does not offer a remedy, or treatment, to the person who suffers from athlete's foot.

It is one object of the present invention to provide a shoe including a medical treatment for athlete's foot. Another object is to provide a bag body containing medicine for insertion into a shoe, or an insole incorporating a bag body.

According to the present invention there is provided a shoe for the medical treatment of athlete's foot characterized by a bag body containing medicine for the treatment of athlete's foot placed inside of the shoe, wherein the medicine is dispersed into the shoe during walking.

With such a shoe the conditions of athlete's foot are treated while walking and the wearer's foot is prevented from being attacked by bacteria or viruses.

Preferably the bag body is board-like in shape and is placed inside the shoe, on the sole thereof so that the medicine for athlete's feet is dispersed into the shoe during the wearer's walking, in use. More preferably the bag body is stored in a hollow portion between the sole and an insole. Even more preferably a plurality of apertures are drilled in the insole and the medicine for athlete's foot is dispersed through the apertures.

Ideally a hollow portion is prepared in advance on the inner bottom of the shoe, into which the bag body is stored while a plurality of apertures are formed in the insole which covers the hollow portion.

Desirably a hard stuffing is provided on the inner bottom of the shoe, a wall prepared all around the stuffing and the insole attached to the wall. More desirably the hollow portion is provided between the inner bottom and the insole, and the bag body is inserted into the hollow portion and a plurality of apertures are formed in the insole so that the medicine for athlete's foot is dispersed into the interior of the shoe, through the apertures.

Ideally the bag body is made of an elastic body composed of porous sponge material. More ideally the medicine is inserted into such a bag body which is of board-like shape.

Desirably the medicine is in the form of a microencapsulated liquid. Alternatively it may be in the form of a powdery solid or a granular solid.

Preferably an extra or separate insole is freely removable and the bag body is attached thereunder with a plurality of preferably drilled apertures being in the extra insole so that the medicine for athlete's foot is dispersed into the shoe.

The invention will now be described, by way of example only, with reference to the accompanying drawings in which:
Figure 1 is a perspective view of a bag body containing medicine for athlete's foot;
Figure 2 is a front cross-sectional view of a shoe of a first embodiment of the present invention;
Figure 3 is a side cross-sectional view of the shoe of the first embodiment;
Figure 4 is a front cross-sectional view of a shoe of a second embodiment of the present invention;
Figure 5 is a front cross-sectional view of a shoe of a third embodiment of the present invention;
Figure 6 is a partial, exploded perspective view of a shoe of the third embodiment;
Figure 7 is a side sectional view of the shoe of the third embodiment;
Figure 8 is a perspective view of an extra insole of a fourth embodiment of the present invention; and
Figure 9 is a cross-sectional view of the insole of Figure 8.

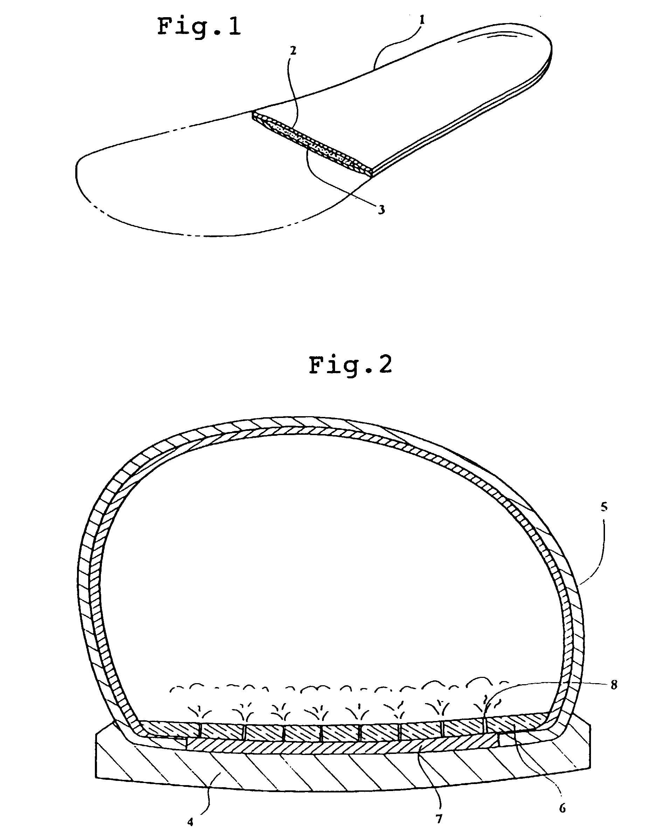

Referring now to Figure 1, this shows a bag body 1 in the form of an elastic body 2 of porous sponge in a board-like shape, with medicine 3 for athlete's foot being sealed therein. The medicine 3 can be dispersed from the bag body 1 through a plurality of apertures in the porous sponge. With regard to thickness of the elastic body 2, various experiments have shown the preferred thickness of the body 2, per sheet, to be about 2mm. The elastic body 2 is composed of two such sheets, and accordingly the overall preferred thickness of the body 2 is 4mm.

It is preferable, in use, that the medicine 3 contained in the bag body 1 effects not only a treatment for athlete's foot, but also treatments for killing/sterilizing bacteria and viruses and deodorizing the feet. The medicine 3 should be easily stored in the bag body 1 and also easily dispersible into the shoe. For example, powdery particles, granular particles or microencapsulated liquid are well suited to this application. If a microencapsulated liquid is adopted, the microcapsule is broken for evaporation by pressure, or alternatively by an increase in temperature or humidity.

In addition to the medicine for athlete's foot, sterilizing or deodorizing agents can be mixed therein, so that a good sanitary condition is maintained and offensive odors are avoided.

When the bag body 1 is placed onto the inside bottom of a shoe, the medicine 3 stored in the bag body 1 is dispersed into the shoe, so that athlete's foot is efficiently treated, as well as sterilization and deodorization. If a microencapsulated liquid is used in the bag body 1, the microcapsule is broken by the pressure of the foot, temperature or humidity, so that the medicine 3 might be slowly and gradually dispersed into the shoe. In this case the dispersal time is maintained for longer than other methods and the remedial effect of the medicine will be accordingly maintained for longer.

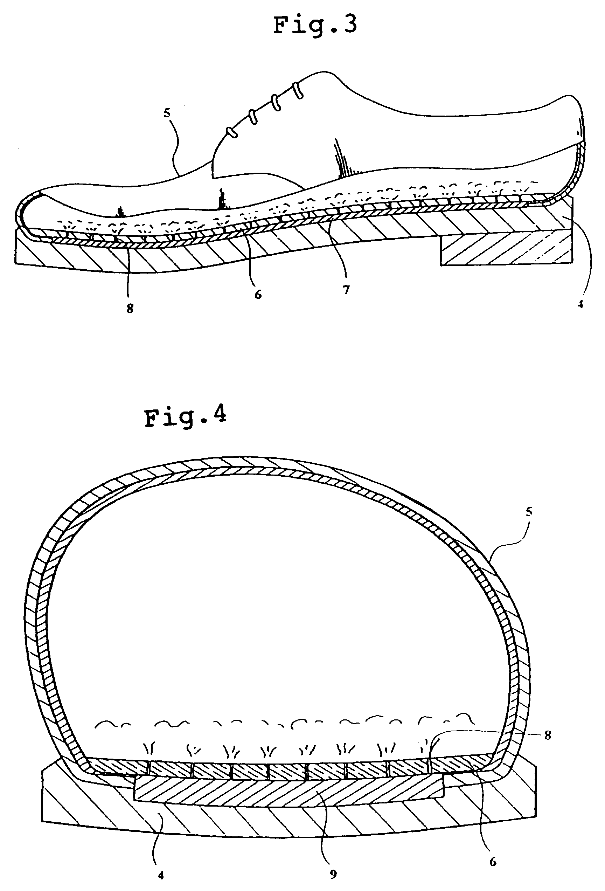

Referring now to Figures 2 and 3, these show a sole 4 of a shoe to which is attached an upper 5 by sewing or other methods. On the inner bottom of the sole 4, an insole 6 is also attached. Between the sole 4 and the insole 6, a hollow portion 7 is formed, into which the bag body 1 is inserted. In order that the medicine 3 stored in the elastic body 2 may be dispersed smoothly into the shoe, a plurality of apertures 8 are provided in the insole 6. The number and position of the apertures 8 can be determined so that the medicine 3 can work effectively over the foot in the shoe.

Referring now to Figure 4, this shows a second embodiment of the present invention with a pre-formed hollow portion 9, into which the bag body 1 is inserted.

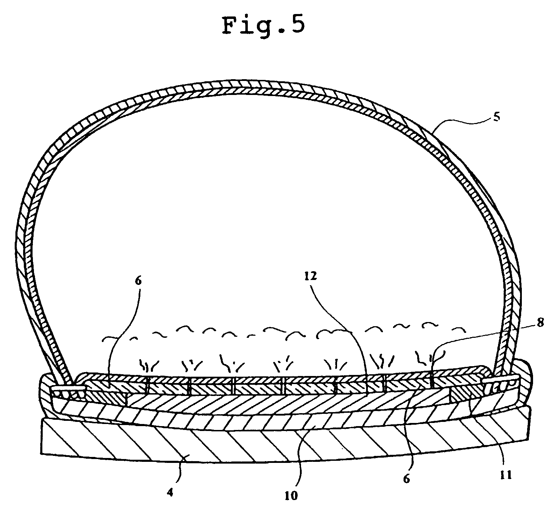

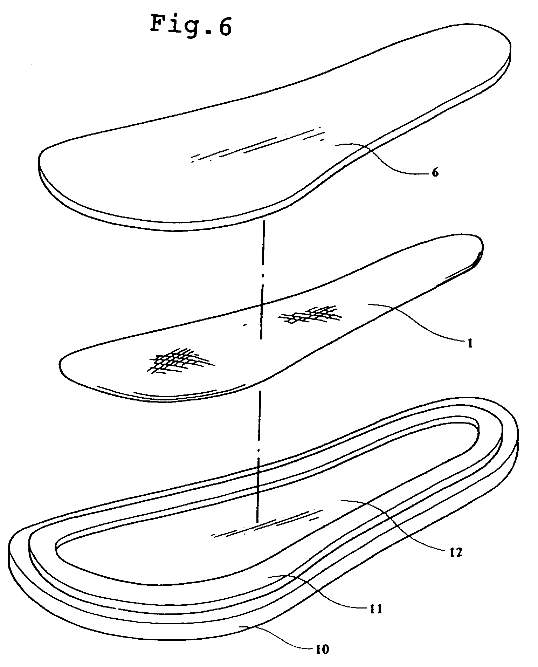

Referring now to Figures 5 to 7, these show a third embodiment of the present invention in which a hard stuffing 10 is attached to the inner surface of the sole 4, and a wall 11 is fixed by an adhesive all around the inside of the shoe, over the stuffing 10. The bag body 1 is inserted into a hollow portion 12 formed between the surface of the stuffing 10 and the wall 11. An insole 6 equipped with a plurality of apertures 8 is attached over the wall 11. An extra or separate insole may be placed over the insole 6. If adopted, the extra insole should be drilled in conformity with the insole 6. Figure 5 shows such an extra insole in place.

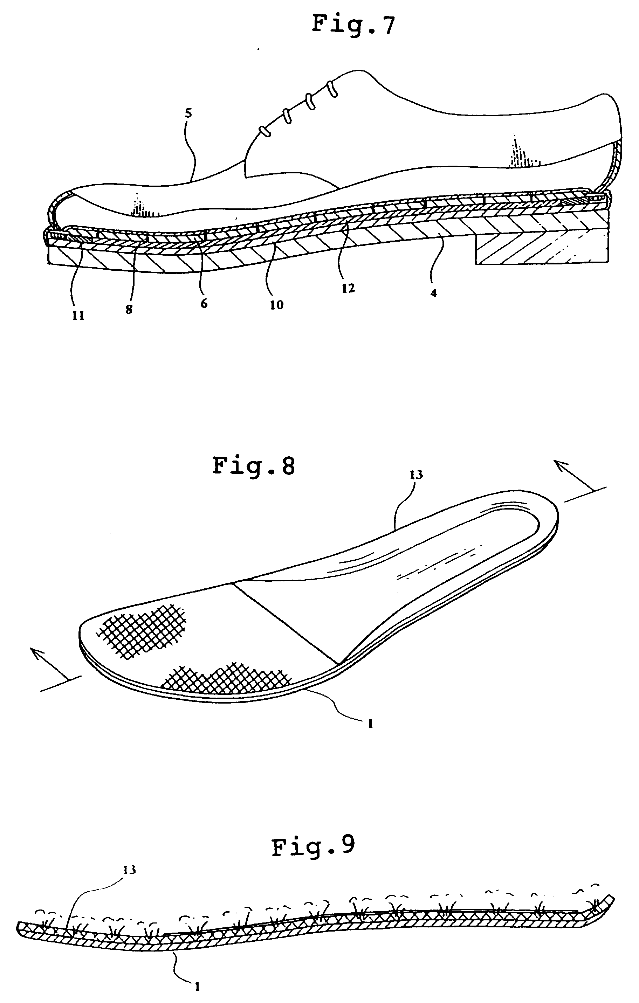

Referring now to Figure 8 and Figure 9, these show, in the form of a fourth embodiment, an extra or separate insole 13. Under the extra insole 13, which is removable, a bag body 1 is attached, with a plurality of drilled apertures being firstly provided in the insole 13. A thin leather cover without apertures may be attached under the bag body 1, so that medicine 3 stored in the bag body 1 will not disperse downward.

Thus in summary, medicine 3 for treating athlete's foot, in the form of a microencapsulated liquid, is stored in a bag body in the form of an elastic body 2 of porous sponge material, the bag body being placed in the shoe and having a plurality of apertures for dispersing the medicine 3 into the shoe. Accordingly when a person suffering from athlete's foot wears such a shoe, the medicine 3 stored in the bag body 1 is gradually dispersed into the shoe during walking, through breaking of the capsule by pressure of the person's weight. Alternatively, the capsule may be broken by body temperature or humidity. Thus, the dispersed medicine 3 treats the skin disease and if sterilizing or deodorizing agents are mixed in the medicine 3, the further effects of preventing bacterial or viral infections and foot odor can also be attained. A further advantage is that as the bag body 1 is placed in the inside of the shoe, no one can tell that the sufferer has athlete's foot as he appears to be wearing normal shoes.

As explained in the fourth embodiment, if the bag body 1 of the present invention is attached to an extra insole and then insert the insole into a shoe, exactly the same remedial effects can be obtained.

## Claims

1. A shoe for the medical treatment of athlete's foot, characterized by a bag body (1) containing medicine (3) for the treatment of athlete's foot placed inside of the shoe, wherein the medicine (3) is dispersed into the shoe during walking.

2. A shoe according to Claim 1, wherein a hollow portion (7) is formed between an inner bottom of the shoe and an insole (6), the bag body (1) being inserted in the hollow portion (7), and a plurality of apertures (8), preferably drilled, are provided in the insole (6) so that the medicine (3) is dispersed into the shoe through the apertures (8), in use.

3. A shoe according to Claim 1, wherein a hollow portion (9) is formed in an inner surface of a sole (4), the bag body (1) being inserted in the hollow portion (9), and an insole (6), which has a plurality of apertures (8), preferably drilled, covers the hollow portion (9) to allow the medicine (3) to disperse through the apertures (8) into the shoe, in use.

4. A shoe according to Claim 1, comprising a stuffing (10) provided on an inner surface of a sole (4), a wall (11) attached around the stuffing (10), an insole (6) covering over the wall (11), and a hollow portion (12) formed between the wall (11) and the inner surface of the sole and the insole cover, the bag body (1) being inserted in the hollow portion (12), a plurality of apertures, preferably drilled, being in the insole cover, wherein the medicine (3) is dispersed into the shoe through the apertures, in use.

5. A shoe according to any one of Claims 1 to 4, wherein the bag body (1) is made of an elastic body (2) composed of a porous sponge material.

6. A shoe according to any one of Claims 1 to 5, wherein medicine (3) contained in the bag body (1) is in a microencapsulated liquid form.

7. A shoe according to any one of Claims 1 to 5, wherein medicine (3) contained in the bag body (1) is in the form of powdery particles.

8. A shoe according to any one of Claims 1 to 5, wherein medicine (3) contained in the bag body (1) is in the form of granular particles.

9. A shoe according to any one of Claims 1 to 8, wherein sterilizing agents are mixed into the medicine (3).

10. A shoe according to any one of Claims 1 to 8, wherein deodorizing agents are mixed into the medicine (3).

11. A bag body (1) containing medicine (3) for the treatment of athlete's foot, comprising an elastic body (2) composed of porous sponge material and the medicine (3) is provided by a liquid containing microcapsule.

12. A separate insole (13), which is freely insertable into and removable from a shoe, comprising a bag body (1) attached on the insole, and a plurality of apertures formed in the insole (13), whereby medicine (3) is dispersed into the shoe, in use
